# EUROPEAN PATENT SPECIFICATION

(11) **EP 4 029 861 B1**
(45) Date of publication and mention of the grant of the patent: **04.11.2026**
(21) Application number: 20861078.2
(22) Date of filing: 04.09.2020
(51) Int. Cl.: C07D 401/14, A61K 31/506, A61P 35/00, A61P 25/28

(54) **METHOD FOR PRODUCING PYRIMIDINYL BIPYRIDINE COMPOUND AND INTERMEDIATE THEREFOR**
VERFAHREN ZUM PRODUZIEREN EINER PYRIMIDINYL-BIPYRIDIN-VERBINDUNG UND ZWISCHENPRODUKT DAFÜR
PROCÉDÉ DE PRODUCTION D'UN COMPOSÉ DE BIPYRIDINE PYRIMIDINYLE ET INTERMÉDIAIRE POUR CELUI-CI

(30) Priority: 06.09.2019 KR 20190110734
(43) Date of publication of application: 20.07.2022
(73) Proprietor: Beyondbio Inc., Daejeon 34054 (KR)
(72) Inventor: MIN, Changhee, Daejeon 34048 (KR); NAH, Ki-Hyun, Cheongju-si, Chungcheongbuk-do 28606 (KR); SON, Hoon Young, Daejeon 35231 (KR)
(74) Representative: V.O.
(86) International application number: PCT/KR2020/011996
(87) International publication number: WO 2021/045585

(56) References cited:
- WO-A1-2018/136700
- WO-A1-2019/032458
- WO-A2-2004/084824
- KR-A- 20040 026 657
- KR-A- 20160 096 033

## Description

### [Technical Field]

The present invention relates to a process for preparing a pyrimidinyl bipyridine compound and an intermediate therefor. More particularly, the present invention relates to a process for preparing a pyrimidinyl bipyridine compound having high purity with high yields without using high-cost silica gels, and an intermediate used therein.

### [Background Art]

The pyrimidinyl bipyridine compound represented by the following formula (1) is a cyclin-dependent kinase (CDK) inhibitor, which is known to be effective in the treatment of cancers such as colon cancer, lung cancer, glioma, and degenerative brain diseases such as Alzheimer's disease [Korean Patent No. 10-1783642].

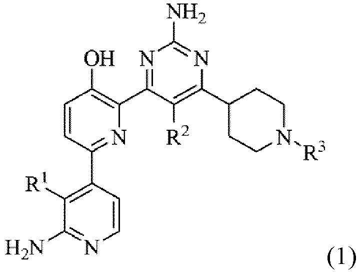

Korean Patent No. 10-1783642 discloses a process for preparing the pyrimidinyl bipyridine compound of formula (1) by reacting the diketone compound of the following formula (2) with guanidine carbonate to obtain a pyrimidinyl pyridine compound, and subjecting it to Suzuki reaction with the dioxaborolane compound of the following formula (3), followed by O-demethylation, as shown in the following reaction scheme 1.

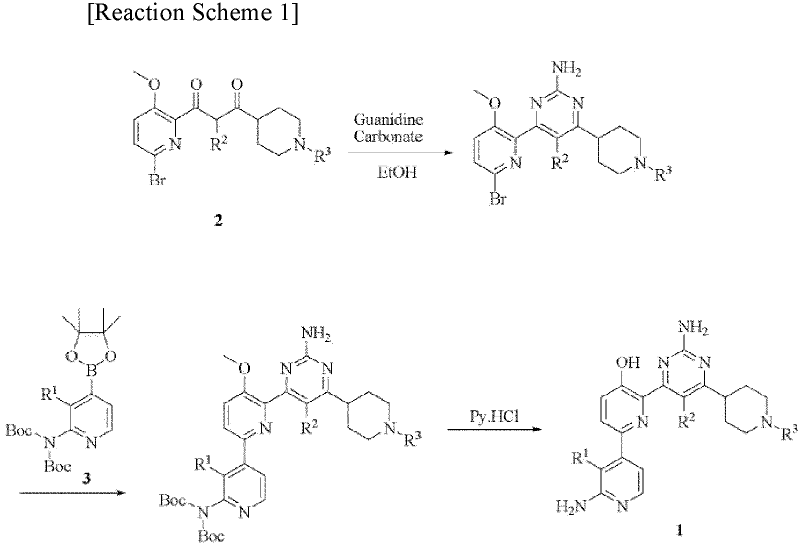

However, the above process has problems that the intermediate, pyrimidinyl pyridine compound has low solubility in organic solvents, so that the yield is significantly lowered during work-up and silica gel column purification processes, and impurities are generated in the O-demethylation process, resulting in lowering the purity.

### [Disclosure]

### [Technical Problem]

The present inventors have studied diligently to solve the above problems in the preparation of the pyrimidinyl bipyridine compound represented by formula (1), and as a result, found out that, in the case that the diketone compound of formula (2) is subjected to Suzuki reaction with the dioxaborolane compound of formula (3), an intermediate having high solubility is generated and a separate O-demethylation process can be omitted. The present invention has been completed therethrough.

Therefore, an object of the present invention is to provide a process for preparing a pyrimidinyl bipyridine compound having high purity with high yields without using high-cost silica gels.

Another object of the present invention is to provide an intermediate used in the preparation process.

### [Technical Solution]

One embodiment of the present invention relates to a process for preparing a pyrimidinyl bipyridine compound of the following formula (1), comprising the steps of:
(i) subjecting a compound of the following formula (2) to Suzuki reaction with a compound of the following formula (3) to obtain a compound of the following formula (4);
(ii) deprotecting the compound of the following formula (4) to obtain a compound of the following formula (5); and
(iii) reacting the compound of the following formula (5) with a guanidine compound: 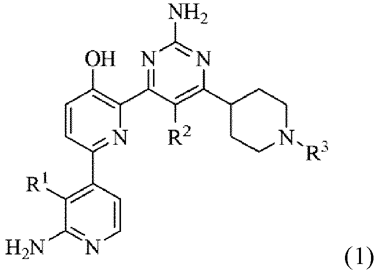 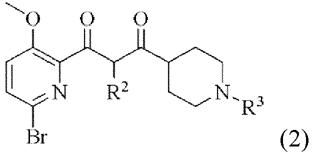 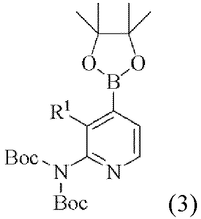 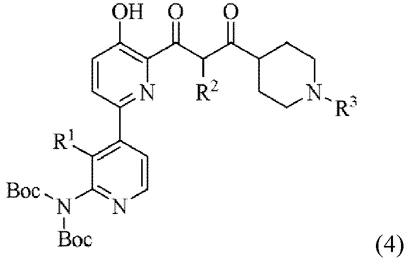 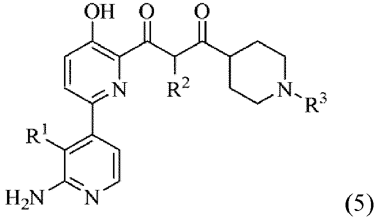 wherein,
   R¹ is halogen,
   R² is hydrogen or a C₁-C₆ alkyl group, and
   R³ is a C₁-C₆ alkyl group.

The term "C₁-C₆ alkyl group" as used herein means a linear or branched hydrocarbon having 1 to 6 carbon atoms, which includes methyl, ethyl, n-propyl, i-propyl, n-butyl, i-butyl, n-pentyl, i-pentyl, n-hexyl and the like, but is not limited thereto.

Hereinafter, the preparation process of the present invention is described in more detail referring to the following reaction scheme 2. The process depicted in the following reaction scheme 2 represents merely a typical example, and various changes may be made to reagents and reaction conditions without limitation.

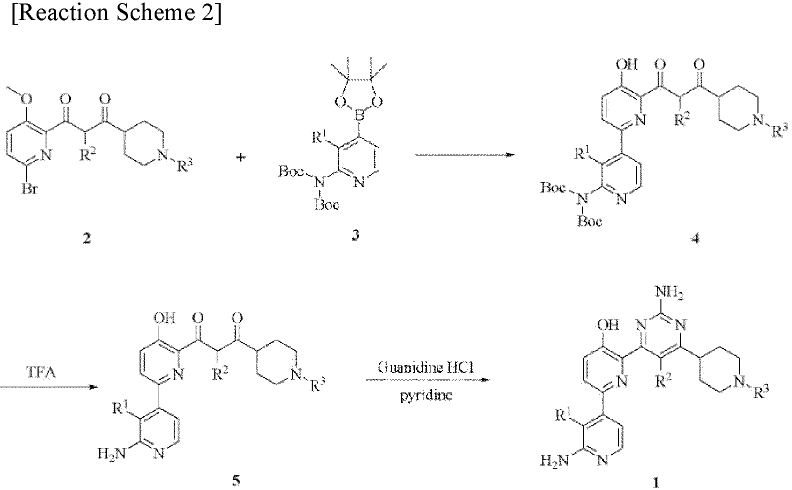

### Step 1: Synthesis of Compound of Formula (4)

The compound of formula (4) can be obtained by subjecting the compound of formula (2) to Suzuki reaction with the compound of formula (3).

The Suzuki reaction may be carried out in the presence of a palladium catalyst and a base.

As the palladium catalyst, tetrakis(triphenylphosphine)palladium(0) (Pd(PPh₃)₄), (1,1'-bis(diphenylphosphino)ferrocene)palladium(II)dichloride (PdCl₂(dppf)₂) and the like may be used, and as the base, potassium phosphate (K₃PO₄), potassium carbonate (K₂CO₃) and the like may be used.

As the reaction solvent, a mixed solvent of tetrahydrofuran and water may be preferably used, and reflux condition is suitable for the reaction temperature.

### Step 2: Synthesis of Compound of Formula (5)

The compound of formula (5) can be obtained by deprotecting the compound of formula (4).

The deprotection may be carried out in the presence of an acid.

As the acid, trifluoroacetic acid (TFA), hydrochloric acid (c-HCl) and the like can be used.

As the reaction solvent, dichloromethane, tetrahydrofuran, acetonitrile and the like may be preferred, and the suitable temperature for the reaction may be - 20°C to room temperature.

### Step 3: Synthesis of Compound of Formula (1)

The compound of formula (1) can be obtained by reacting the compound of formula (5) with a guanidine compound.

The guanidine compound may be guanidine HCl, guanidine carbonate and the like.

The reaction may be carried out in the presence of a base in pyridine solvent.

As the base, potassium carbonate (K₂CO₃), cesium carbonate (Cs₂CO₃) and the like may be used.

In the reaction, reflux condition is suitable for the reaction temperature.

The compound of formula (1) prepared as above can be purified with acetone and ethanol to obtain the compound having a purity of 99% or higher.

One embodiment of the present invention relates to a process for preparing a pharmaceutically acceptable salt of the compound of formula (1) by reacting the compound of formula (1) prepared by the above preparation process with an acid.

As the acid, non-toxic inorganic and organic acids may be used, and examples thereof may include hydrochloric acid, phosphoric acid, sulfuric acid, nitric acid, tartaric acid, methanesulfonic acid, p-toluenesulfonic acid, acetic acid, trifluoroacetic acid, citric acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid, malic acid, malonic acid and the like. Particularly, hydrochloric acid is preferred.

As the reaction solvent, methanol, 1,4-dioxane and the like are preferred, and the suitable reaction temperature is room temperature.

The pharmaceutically acceptable salt of the compound of formula (1) prepared as above can be recrystallized with water and acetone to obtain the compound having a purity of 99% or higher.

One embodiment of the present invention relates to a compound of formula (4) or a compound of formula (5) which is an intermediate for preparing the compound of formula (1): 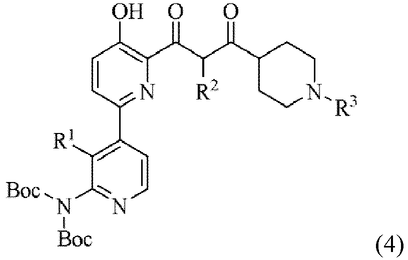 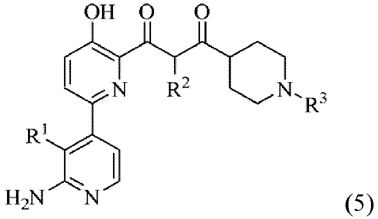 wherein,
R¹ is halogen,
R² is hydrogen or a C₁-C₆ alkyl group, and
R³ is a C₁-C₆ alkyl group.

In one embodiment of the present invention, R¹ is fluorine, R² is methyl group, and R³ is i-propyl group.

### [Advantageous Effects]

In accordance with the preparation process of the present invention, by using the intermediate of formula (4) having excellent solubility thereby facilitating work-up and purification, the pyrimidinyl bipyridine compound of formula (1) having high purity can be mass-produced with high yields without using high-cost silica gels.

### [Best Mode]

Hereinafter, the present invention will be described in more detail by the following examples. It will be obvious to those skilled in the art that these examples are merely described for illustration of the present invention and the scope of the present invention is not limited thereto.

### Preparation Example 1: Preparation of 2-[3-fluoro-4-(4,4,5,5-tetramethyl-[1,3,2]dioxaborolan-2-yl)-2-pyridinyl]-imidodicarbonic acid-1,3-bis(1,1-dimethylethyl) ester

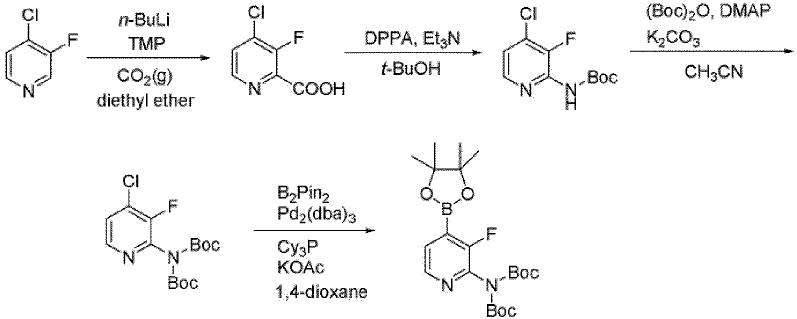

### Preparation Example 1-1: Preparation of 4-chloro-3-fluoro-pyridine-2-carboxylic acid

The inside of the reactor was filled with nitrogen, and 2.5M *n*-butyllithium hexane solution (*n*-BuLi (2.5M in Hexane)) (502 mL, 1.254 mol) was added to diethylether at -78°C. 2,2,6,6-Tetramethylpiperidine (TMP) (213 mL, 1.254 mol) was added dropwise thereto, followed by stirring for 1 hour. 4-Chloro-3-fluoropyridine (150 g, 1.140 mol) was slowly added dropwise and stirred at -78°C for 1 hour. The resulting reaction solution was slowly added dropwise to a reactor having diethyl ether with a large number of crushed dry ice. After the dropwise addition, the temperature was slowly raised to room temperature, and the reaction solution was concentrated. A 2N hydrochloric acid aqueous solution was slowly added to form a solid, followed by stirring for 30 minutes and filtration. The solid was washed with water, and *n*-hexane was added to the filtered solid compound, followed by stirring for 30 minutes and filtration. After washing with *n*-hexane, the solid compound was dried in a dryer at 65°C for 16 hours. The dried compound was purified with *n*-hexane/diethyl ether and dried to obtain the white title compound (150 g, 75%).

¹H NMR (600 MHz, DMSO-*d*₆) *δ* 8.45 (d, *J* = 5.4 Hz, 1H), 7.93 (t, *J =* 5.4 Hz, 1H)

### Preparation Example 1-2: Preparation of (4-chloro-3-fluoro-pyridin-2-yl)-carbamic acid-tert-butyl ester

To a reactor were added 4-chloro-3-fluoro-pyridine-2-carboxylic acid (1.23 kg, 7.007 mol) and *tert*-butanol. Triethylamine (2.3 L, 16.116 mol) was added thereto, and diphenylphosphoryl azide (DPPA) (2.3 L, 10.511 mol) was added, followed by raising the temperature. The reaction was performed under reflux condition for 16 hours, and then the temperature was lowered to room temperature, followed by concentration under reduced pressure. 1N hydrochloric acid aqueous solution and ethyl acetate were added thereto, followed by stirring for 1 hour. The organic layer was separated, and pH was neutralized with saturated sodium hydrogen carbonate aqueous solution. The organic layer was separated, and magnesium sulfate (MgSO₄) and activated carbon were added thereto, followed by stirring for 1 hour to improve the moisture and color of the organic layer. It was filtered with celite, washed with ethyl acetate, and concentrated under reduced pressure to obtain the light brown title compound (1.73 kg, Quant.).

¹H NMR (600 MHz CDCl₃) *δ* 8.11 (d, *J =* 4.8 Hz, 1H), 7.09 (br, 1H), 7.08 (t, *J* = 4.8 Hz, 1H), 1.52 (s, 9H)

### Preparation Example 1-3: Preparation of 2-(3-fluoro-4-chloropyridinyl)-imidocarbonic acid-1,3-bis(1,1-dimethylethyl)ester

(4-Chloro-3-fluoro-pyridin-2-yl)-carbamic acid-*tert*-butyl ester (1.723 kg, 6.987 mol) was dissolved in acetonitrile, and di-*tert*-butyl dicarbonate ((Boc)₂O) (3.05 kg, 13.784 mol) and potassium carbonate (K₂CO₃) (2.9 kg, 20.961 mol) were added thereto and stirred. After slowly adding 4-dimethylaminopyridine (85 g, 0.699 mol), the resulting mixture was stirred at room temperature for 16 hours. After filtration with celite, it was washed with ethyl acetate and concentrated under reduced pressure. 1N hydrochloric acid aqueous solution and ethyl acetate were added and stirred for 1 hour, and then the organic layer was separated. A saturated sodium hydrogen carbonate aqueous solution was added to the organic layer to adjust pH to 8 and stirred for 1 hour. The organic layer was separated, and magnesium sulfate (MgSO₄) and activated carbon were added to improve the moisture and color of the organic layer. After filtration with celite, it was washed with ethyl acetate and concentrated under reduced pressure. After adding *n-*hexane/diethyl ether, the resulting mixture was cooled to 0 ~ 4 °C and stirred for recrystallization. The resulting solid was filtered, washed with n-hexane and dried to obtain the light brown title compound (1.46 kg, 60%).

¹H NMR (600 MHz CDCl₃) *δ* 8.20 (d, *J =* 5.4 Hz, 1H), 7.36 (t, *J =* 4.8 Hz, 1H), 1.43 (s, 18H)

### Preparation Example 1-4: Preparation of 2-[3-fluoro-4-(4,4,5,5-tetramethyl-[1,3,2]dioxaborolan-2-yl)-2-pyridinyl]-imidodicarbonic acid-1,3-bis(1,1-dimethylethyl)ester

2-(3-Fluoro-4-chloropyridinyl)-imidocarbonic acid-1,3-bis(1,1-dimethylethyl)ester (150 g, 0.433 mol) was dissolved in 1,4-dioxane. Then, bis(pinacolato)diboron (241 g, 0.952 mol), tris(dibenzylideneacetone)dipalladium (19.8 g, 0.0216 mol), tricyclohexylphosphine (9.0 g, 0.0344 mol), and potassium acetate (170 g, 1.730 mol) were added, followed by stirring under reflux for 16 hours. After the reaction was completed, it was cooled to room temperature, and celite and dichloromethane were added to the reactor, followed by stirring for 30 minutes. It was filtered with celite to remove the solid residue and washed with dichloromethane. The filtrate was concentrated under reduced pressure, dichloromethane was added, sodium hydrogen carbonate aqueous solution was added, and the resulting mixture was stirred for 30 minutes. At this time, the pH of the water layer should be basic. The organic layer was separated, and magnesium sulfate (MgSO₄) and activated carbon were added to improve the moisture and color of the organic layer. After filtration with celite, it was washed with dichloromethane and concentrated under reduced pressure. Then, *n*-hexane was added and recrystallization was performed to obtain the white title compound (133 g, 70%).

¹H NMR (600 MHz CDCl₃) *δ* 8.29 (d, *J =* 4.8 Hz, 1H), 7.57 (t, *J =* 4.8 Hz, 1H), 1.39 (s, 18H), 1.34 (s, 12H)

### Preparation Example 2: Preparation of methyl 6-bromo-3-methoxypicolinate

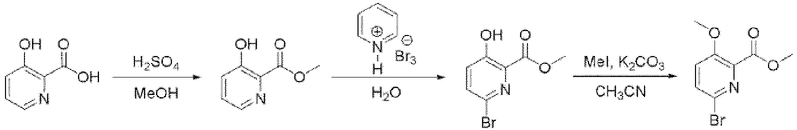

### Preparation Example 2-1: Preparation of methyl 3-hydroxypicolinate

After 3-hydroxypicolinic acid (2.4 kg, 17.32 mol) was added to methanol, sulfuric acid (2.4 L) was slowly added dropwise at 0°C and stirred under reflux for 24 hours. After cooling to room temperature, it was concentrated under reduced pressure. After adding water and cooling to 0°C, pH was adjusted to 7 ~ 8 using 6N sodium hydroxide aqueous solution. After extraction with ethyl acetate, magnesium sulfate (MgSO₄) was added, and the organic layer was dried and concentrated under reduced pressure. Then, n-hexane was added, and recrystallization was performed to obtain the off-white title compound (2.0 kg, 75%).

¹H NMR (600 MHz, CDCl₃) *δ* 10.63 (s, 1H), 8.28 (s, 1H), 7.41 (t, *J* = 1.8 Hz, 1H), 7.37 (d, *J =* 1.8 Hz, 1H), 4.06 (s, 3H)

### Preparation Example 2-2: Preparation of methyl 6-bromo-3-hydroxypicolinate

Methyl 3-hydroxypicolinate (1.2 kg, 7.833 mol) was dissolved in distilled water (30 L), and then pyridinium tribromide (3.24 kg, 10.185 mol) was slowly added dropwise thereto. After completion of the addition, the resulting mixture was stirred at room temperature for 24 hours. The resulting solid was filtered and washed with water thoroughly. The filtered solid was dissolved in dichloromethane, the organic layer was separated, and magnesium sulfate (MgSO₄) was added. The organic layer was dried, concentrated under reduced pressure, and vacuum-dried to obtain the off-white title compound (1.46 kg, 80%).

¹H NMR (600 MHz CDCl₃) *δ* 10.69 (s, 1H), 7.56 (d, *J =* 8.4 Hz, 1H), 7.27 (d, *J =* 8.4 Hz, 1H), 4.06 (s, 3H)

### Preparation Example 2-3: Preparation of methyl 6-bromo-3-methoxypicolinate

Methyl 6-bromo-3-hydroxypicolinate (230 g, 0.9912 mol) was added to acetonitrile. Potassium carbonate (274 g, 1.9825 mol) and methane iodide (123 mL, 1.9825 mol) were added thereto, and the temperature was raised. After stirring under reflux condition for 16 hours and cooling to room temperature, it was filtered with celite. After washing with ethyl acetate, the filtrate was concentrated under reduced pressure. The organic layer was extracted from the concentrate using water and dichloromethane and separated, and magnesium sulfate (MgSO₄) was added. The organic layer was dried and concentrated under reduced pressure. The concentrate was recrystallized with n-hexane/diethyl ether and washed with n-hexane to obtain the off-white title compound (190 g, 78%).

¹H NMR (600 MHz CDCl₃) *δ* 10.69 (s, 1H), 7.57 (d, *J =* 8.4 Hz, 1H), 7.26 (d, *J =* 8.4 Hz, 1H), 3.95 (s, 3H), 3.91 (s, 3H)

### Preparation Example 3: Preparation of 1-(1-isopropylpiperidin-4-yl)propan-1-one

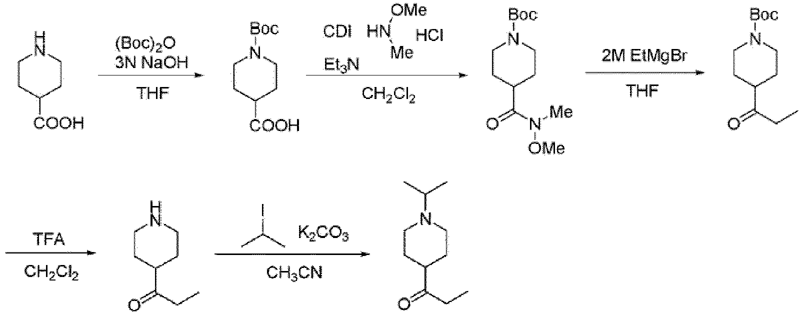

### Preparation Example 3-1: Preparation of 1-(tert-butoxycarbonyl)piperidine-4-carboxylic acid

4-Piperidinecarboxylic acid (70.0 g, 0.542 mol) was dissolved in tetrahydrofuran (THF), and then di-*tert*-butyl dicarbonate ((Boc)₂O) (236.6 g, 1.084 mol) and 3N sodium hydroxide aqueous solution were added. After stirring at room temperature for 16 hours, completion of the reaction was confirmed, and the reaction solution was concentrated under reduced pressure. 3N hydrochloric acid aqueous solution was added to the concentrate to adjust pH to 7 ~ 8. Dichloromethane was added and the organic layer was separated. The separated organic layer was dried over magnesium sulfate (MgSO₄) and filtered. After the filtrate was concentrated under reduced pressure, *n*-hexane was added for recrystallization to obtain the white title compound (121.8 g, 98.0%).

¹H NMR (600 MHz, CDCl₃) *δ* 4.02 (br, 2H), 2.86 (br, 2H), 2.48 (m, 1H), 1.91 (br, 2H), 1.63 (m, 2H), 1.45 (s, 9H)

### Preparation Example 3-2: Preparation of tert-butyl-4-(methoxy(methyl)carbamoyl)piperidine-1-carboxylate

After dissolving 1-(*tert*-butoxycarbonyl)piperidine-4-carboxylic acid (118 g, 0.515 mol) in dichloromethane, 1,1'-carbonyldiimidazole (CDI) (108.6 g , 0.669 mol) was added. After the addition was completed, the resulting mixture was stirred at room temperature for 1 ~ 2 hours. N,O-dimethylhydroxylamine hydrochloride (65.3 g, 0.669 mol) and triethylamine (Et₃N) (67.7 g, 0.669 mol) were added and stirred at room temperature for 16 hours. After confirming completion of the reaction, saturated ammonium chloride (Sat. NH₄Cl) was added, and the organic layer was separated. To the separated organic layer was added 2N hydrochloric acid aqueous solution, followed by separation of the organic layer. Saturated sodium chloride (Brine) was added to the separated organic layer and the organic layer was separated. The separated organic layer was dried over magnesium sulfate (MgSO₄) and filtered. The filtrate was concentrated under reduced pressure to obtain the white title compound (137.8 g, 95.3%).

¹H NMR (600 MHz, CDCl₃) *δ* 4.12 (br, 2H), 3.71 (s, 3H), 3.18 (s, 3H), 2.79 (br, 3H), 1.63 (m, 4H), 1.46 (s, 9H)

### Preparation Example 3-3: Preparation of tert-butyl 4-propionyl piperidine-1-carboxylate

*tert*-Butyl-4-(methoxy(methyl)carbamoyl)piperidine-1-carboxylate (132 g, 0.485 mol) was dissolved in tetrahydrofuran (THF), and then the temperature was lowered to 4 ~ 10°C. 2M ethylmagnesium bromide (2M EtMgBr) (727 mL, 1.454 mol) was slowly added and stirred for 30 minutes, followed by stirring at room temperature for 16 hours. After confirming completion of the reaction, it was cooled to 4 ~ 10°C, and water was slowly added thereto. 2N hydrochloric acid aqueous solution was added, and the organic layer was separated. The separated organic layer was dried over magnesium sulfate (MgSO₄) and filtered. The filtrate was concentrated under reduced pressure to obtain the yellow title compound (109 g, 93%).

¹H NMR (600 MHz, CDCl₃) *δ* 4.11 (br s, 2H), 2.77 (s, 2H), 2.52-2.42 (m, 3H), 1.79 (br s, 2H), 1.58-1.48 (m, 2H), 1.45 (s, 9H), 1.05 (t, *J=* 7.2 Hz, 3H)

### Preparation Example 3-4: Preparation of 1-(piperidin-4-yl)propan-1-one

*tert*-Butyl 4-propionyl piperidine-1-carboxylate (130 g, 0.539 mol) was dissloved in dichloromethane, and trifluoroacetic acid (TFA) (301 mL, 3.932 mol) was slowly added, followed by stirring at room temperature for 16 hours. After confirming completion of the reaction, the reaction solution was concentrated under reduced pressure. To the concentrate were added 3N sodium hydroxide aqueous solution and dichloromethane, and the organic layer was separated. The separated organic layer was dried over magnesium sulfate (MgSO₄) and filtered. The filtrate was concentrated under reduced pressure to obtain the brown title compound (68.4 g, 90%).

¹H NMR (600MHz, CDCl₃) *δ* 3.12 (d, J = 10.8 Hz 2H), 2.63 (t, J = 12 Hz, 2H), 2.48-2.42 (m, 3H), 1.80 (d, J = 13.2 Hz, 2H), 1.62 (s, 2H), 1.54-1.48 (m, 2H), 1.06-1.03 (m, 3H)

### Preparation Example 3-5: Preparation of 1-(1-isopropylpiperidin-4-yl)propan-1-one

1-(Piperidin-4-yl)propan-1-one (76 g, 0.538 mol) was dissolved in acetonitrile, and then potassium carbonate (K₂CO₃) (223.1 g, 1.614 mol) and isopropyl iodide (137.2 g, 0.807 mol) were added. The temperature was raised, and the resulting mixture was stirred under reflux condition for 16 hours. After confirming completion of the reaction, the temperature was lowered to room temperature, followed by celite filtration. The filtrate was concentrated under reduced pressure, water and ethyl acetate were added, and the organic layer was separated. The separated organic layer was dried over magnesium sulfate (MgSO₄) and filtered. The filtrate was concentrated under reduced pressure and distilled under high pressure to obtain the colorless title compound (68.4 g, 90%).

¹H NMR (600MHz, CDCl₃) *δ* 2.92-2.86 (m, 2H), 2.74-2.68 (m, 1H), 2.47 (q, J = 7.2 Hz, 2H), 2.32-2.24 (m, 1H), 2.16 (t, J = 11.4 Hz, 2H), 1.87 (d, J = 13.2 Hz, 2H), 1.70-1.60 (m, 2H), 1.08-1.02 (m, 9H)

### Preparation Example 4: Preparation of 1-(6-bromo-3-methoxypyridin-2-yl)-3-(1-isopropylpiperidin-4-yl)-2-methylpropane-1,3-dione (2a)

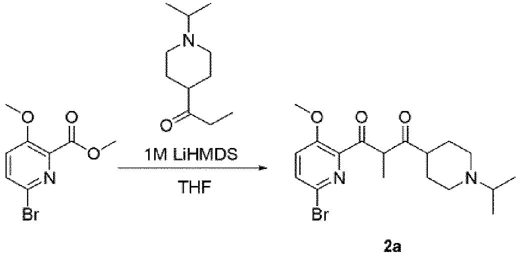

Methyl 6-bromo-3-methoxypicolinate (615.15 g, 2.50 mol) obtained from Preparation Example 2 was dissolved in tetrahydrofuran (THF), and then 1-(1-isopropylpiperidin-4-yl)propan-1-one (549.9 g, 3.00 mol) obtained from Preparation Example 3 was added. The temperature was lowered to -70 ~ -78°C, and 1M lithium bis(trimethylsilyl)amide (1M LiHMDS) (5.00 L, 5.00 mol) was slowly added. After the addition was completed, the resulting mixture was stirred for 30 minutes, and the temperature was slowly raised to room temperature. After stirring at room temperature for 3 hours, when the content of methyl 6-bromo-3-methoxypicolinate was 5 ~ 7% by HPLC, saturated ammonium chloride (Sat. NH₄Cl) was slowly added to terminate the reaction. The organic layer was separated, and the separated organic layer was dried over magnesium sulfate (MgSO₄) and filtered. The filtrate was concentrated under reduced pressure to obtain the light brown title compound (537 g, yield 54%, purity >95%).

¹H NMR (600MHz, CDCl₃) δ 7.54 (d, J = 9 Hz, 1H), 7.27 (d, J = 9 Hz, 1H), 4.82-4.70 (m, 1H), 3.92 (s, 3H), 2.94 (d, J = 9 Hz, 2H), 2.85 (br s, 1H), 2.61 (br s, 1H), 2.40-2.22 (m, 2H), 2.10-1.90 (m, 2H), 1.80-1.60 (m, 4H), 1.35 (d, J = 7.2 Hz, 3H), 1.05 (d, J = 5.4 Hz, 6H)

### Example 1: Preparation of 2'-amino-6-(2-amino-6-(1-isopropylpiperidin-4-yl)-5-methylpyrimidin-4-yl)-3'-fluoro-[2,4'-bipyridin]-5-ol (1a)

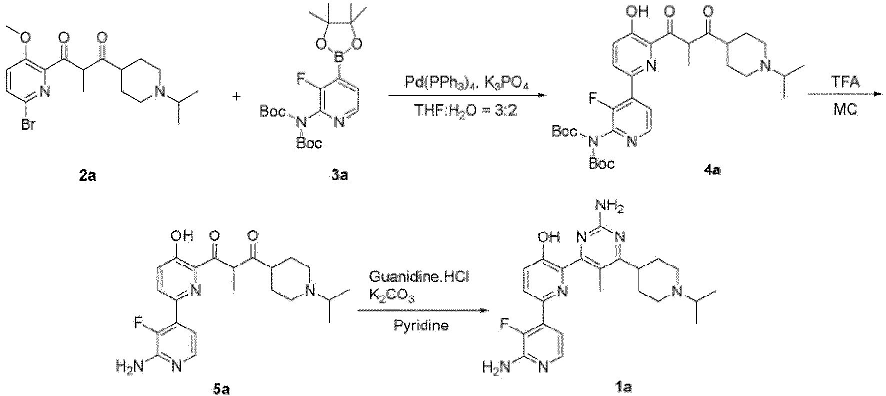

### Example 1-1: Preparation of tert-Butyl (3'-fluoro-5-hydroxy-6-(3-(1-isopropylpiperidin-4-yl)-2-methyl-3-oxopropanoyl)-[2,4'-bipyridin]-2'-yl) dicarbamate (4a)

1-(6-Bromo-3-methoxypyridin-2-yl)-3-(1-isopropylpiperidin-4-yl)-2-methylpropane-1,3-dione (2a) (795 g, 2.00 mol) obtained from Preparation Example 4, 2- [3-fluoro-4-(4,4,5,5-tetramethyl-[1,3,2]dioxaborolan-2-yl)-2-pyridinyl]-imidodicarbonic acid-1,3-bis(1,1-dimethylethyl)ester (1.23 kg, 2.80 mol) obtained from Preparation Example 1, tetrakis(triphenylphosphine) palladium(0) (Pd(PPh₃)₄) (115.6 g, 0.10 mol) and potassium phosphate (K₃PO₄) (1.27 kg, 6.00 mol) were added to tetrahydrofuran/water (3/2). The temperature was raised, and the resulting mixture was stirred under reflux for 16 hours. After completion of the reaction, it was cooled to room temperature. After the organic layer was separated, saturated sodium chloride (NaCl) aqueous solution was added to the organic layer, and the organic layer was re-separated. The separated organic layer was dried over magnesium sulfate (MgSO₄) and filtered. The filtrate was concentrated under reduced pressure, and potassium phosphate (K₃PO₄) (1.27 kg, 6.00 mol) and tetrahydrofuran/water (3/2) were added to the concentrated solution. The temperature was raised, and the resulting mixture was stirred under reflux condition for 16 hours. After completion of the reaction, it was cooled to room temperature. The organic layer was separated, saturated sodium chloride (NaCl) aqueous solution was added to the organic layer, and the organic layer was re-separated. The separated organic layer was dried over magnesium sulfate (MgSO₄) and filtered. The filtrate was concentrated under reduced pressure to obtain the title compound (860 g, yield 70%, purity > 95%).

¹H NMR (600MHz, CDCl₃) δ 8.41 (d, J = 5.4 Hz, 1H), 8.21 (t, J = 5.4 Hz, 1H), 8.16 (d, J = 7.8 Hz, 1H), 7.90 (d, J = 7.8 Hz, 1H), 3.09 - 3.11 (m, 2H), 2.92 - 2.96 (m, 1H), 2.83 - 2.85 (m, 1H), 2.31 - 2.34 (m, 2H), 2.13 - 2.18 (m, 5H), 1.87 - 1.90 (m, 2H), 1.43 (s, 18H), 1.12 (d, J = 6.6 Hz, 6H)

### Example 1-2: Preparation of 1-(2'-amino-3'-fluoro-5-hydroxy-[2,4'-bipyridin]-6-yl)-3-(1-isopropylpiperidin-4-yl)-2-methylpropane-1,3-dione (5a)

*tert*-Butyl (3'-fluoro-5-hydroxy-6-(3-(1-isopropylpiperidin-4-yl)-2-methyl-3-oxopropanoyl)-[2,4'-bipyridin]-2'-yl) dicarbamate (4a) (20 g, 0.03 mol) was dissolved in dichloromethane, the temperature was lowered to -10 ~ 0 °C, and trifluoroacetic acid (TFA) (37.4 mL, 0.49 mol) was slowly added. When the addition was completed, the resulting mixture was stirred at room temperature for 16 hours. After completion of the reaction, the reaction solution was concentrated under reduced pressure. To the concentrate were added water and ethyl acetate, and pH was adjusted to 12 ~ 14 with 2N sodium hydroxide aqueous solution. The resulting solid was filtered to obtain the brown title compound (9.4 g, yield 70%, purity > 98%).

¹H NMR (600MHz, DMSO-d₆) δ 8.23 (d, J = 9 Hz, 1H), 8.11 (d, J = 9 Hz, 1H), 7.84 (d, J = 5.4 Hz, 1H), 6.96 (t, J = 4.8 Hz, 1H), 6.40 (br s, 2H), 2.86 - 2.91 (m, 3H), 2.69 - 2.71 (m, 1H), 2.20 - 2.24 (m, 2H), 2.01 (s, 3H), 1.85 - 1.87 (m, 2H), 1.75 - 1.77 (m, 2H), 0.96 (d, J = 6.6 Hz, 6H)

### Example 1-3: Preparation of 2'-amino-6-(2-amino-6-(1-isopropylpiperidin-4-yl)-5-methylpyrimidin-4-yl)-3'-fluoro-[2,4'-bipyridin]-5-ol (1a)

1-(2'-amino-3'-fluoro-5-hydroxy-[2,4'-bipyridin]-6-yl)-3-(1-isopropylpiperidin-4-yl)-2-methylpropane-1,3-dione (5a) (100 g, 0.24 mol), guanidine HCl (230.48 g, 2.41 mol) and potassium carbonate (K₂CO₃) (666.91 g, 4.82 mol) were added to pyridine. The temperature was raised, and the resulting mixture was stirred under reflux condition for 24 hours. After completion of the reaction, the temperature was lowered to 50 ~ 60 °C, followed by filtration. The filtrate was concentrated under reduced pressure and water was added thereto. After the temperature was lowered to -10 ~ -5°C, pH was adjusted to 1 ~ 2 with 2N hydrochloric acid aqueous solution, and the pH was adjusted to 7 ~ 7.5 with 2N sodium hydroxide. The resulting solid was filtered, washed with water, and dried to obtain the title compound (105 g, yield 99.5%).

Then, to purify the title compound obtained above, purification process was performed with acetone and ethanol to obtain the title compound (80.7 g, yield 76.5%, purity > 99%).

¹H NMR (600MHz, DMSO-d₆) δ 7.79 (d, J = 7.8 Hz, 1H), 7.77 (d, J = 5.4 Hz, 1H), 7.46 (d, J = 8.4 Hz, 1H), 6.98 (t, J = 4.8 Hz, 1H), 6.61 (s, 2H), 6.27 (s, 2H), 2.95 (d, J = 11.4 Hz, 2H), 2.92 - 2.86 (m, 1H), 2.84 - 2.76 (m, 1H), 2.35 (t, J = 10.8 Hz, 2H), 2.25 (s, 3H), 1.90 - 1.80 (m, 2H), 1.70 (d, J = 12.6 Hz, 2H), 1.03 (d, J = 6.6 Hz, 6H)

### Example 2: Preparation of 2'-amino-6-(2-amino-6-(1-isopropylpiperidin-4-yl)-5-methylpyrimidin-4-yl)-3'-fluoro-[2,4'-bipyridin]-5-ol trihydrochloride

2'-Amino-6-(2-amino-6-(1-isopropylpiperidin-4-yl)-5-methylpyrimidin-4-yl)-3'-fluoro-[2,4'-bipyridin]-5-ol (1a) (56 g, 0.13 mol) obtained from Example 1 was reacted with hydrochloric acid in methanol and stirred at room temperature for 24 hours. The reaction solution was concentrated under reduced pressure, and water was added to dissolve it. Acetone was slowly added to the dissolved solution to proceed with recrystallization. The resulting solid was filtered, washed with acetone, and dried. To completely remove the residual solvent of the dried solid compound, it was dissolved in water and recrystallized with acetone to obtain the title compound (63 g, yield 90%, purity > 99%).

¹H NMR (600MHz, DMSO-d₆) δ 8.41 (br s, 1H), 8.06 - 8.07 (m, 1H), 7.86 - 7.87 (m, 1H), 7.74 (m, 1H), 7.42 (m, 1H), 3.59 - 3.63 (m, 2H), 3.43 - 3.45 (m, 1H), 3.43 (m, 1H), 3.11 - 3.16 (m, 2H), 2.16 - 2.23 (m, 2H), 2.14 (s, 3H), 1.98 - 2.00 (m, 2H), 1.03 (d, J = 6.6 Hz, 6H)

## Claims

1. A process for preparing a pyrimidinyl bipyridine compound of the following formula (1), which comprises the steps of:
(i) subjecting a compound of the following formula (2) to Suzuki reaction with a compound of the following formula (3) to obtain a compound of the following formula (4);
(ii) deprotecting the compound of the following formula (4) to obtain a compound of the following formula (5); and
(iii) reacting the compound of the following formula (5) with a guanidine compound: 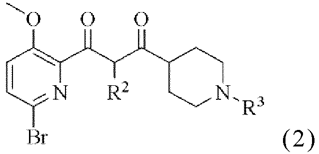 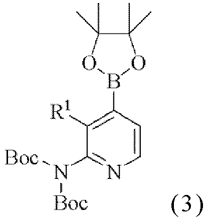 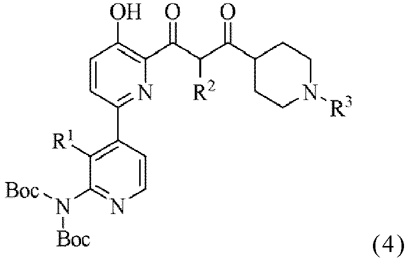 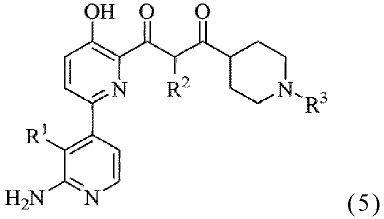 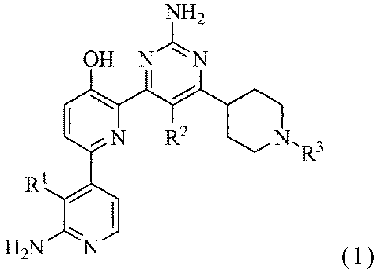 wherein,
R¹ is halogen,
R² is hydrogen or a C₁-C₆ alkyl group, and
R³ is a C₁-C₆ alkyl group.

2. The process according to claim 1, wherein the Suzuki reaction of the step (i) is carried out in the presence of tetrakis(triphenylphosphine)palladium(0) and potassium phosphate.

3. The process according to claim 1, wherein the deprotection of the step (ii) is carried out in the presence of trifluoroacetic acid.

4. The process according to claim 1, wherein the guanidine compound of the step (iii) is guanidine hydrochloride.

5. The process according to claim 1, wherein the reaction of the step (iii) is carried out in the presence of potassium carbonate in pyridine solvent.

6. The process according to claim 1, comprising the step of purifying the pyrimidinyl bipyridine compound of formula (1) obtained from the step (iii) with acetone and ethanol.

7. The process according to any one of claims 1 to 6, comprising the further step of reacting the compound of formula (1) with an acid to form a pharmaceutically acceptable salt of the compound of formula (1).

8. The process according to claim 7, wherein the acid is hydrochloric acid, and the pharmaceutically acceptable salt of the compound of formula (1) is trihydrochloride salt of the compound of formula (1).

9. The process according to claim 8, comprising the step of recrystallizing the trihydrochloride salt of the compound of formula (1) with water and acetone.

10. A compound of the following formula (4): 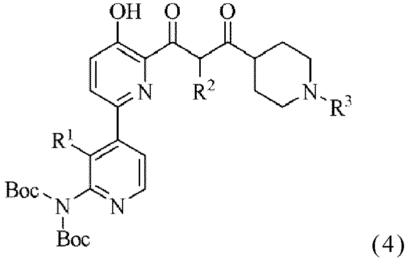 wherein,
R¹ is halogen,
R² is hydrogen or a C₁-C₆ alkyl group, and
R³ is a C₁-C₆ alkyl group.

11. A compound of the following formula (5): 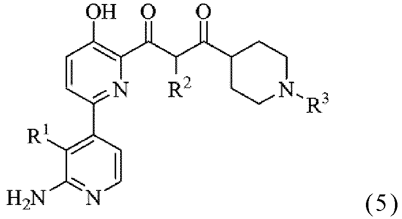 wherein,
R¹ is halogen,
R² is hydrogen or a C₁-C₆ alkyl group, and
R³ is a C₁-C₆ alkyl group.

12. The compound according to claim 10 or 11, wherein R¹ is fluorine, R² is methyl group, and R³ is i-propyl group.

## Patentansprüche

1. Verfahren zur Herstellung einer Pyrimidinyl-Bipyridin-Verbindung der folgenden Formel (1), welches die Schritte umfasst von:
(i) Unterziehen einer Verbindung der folgenden Formel (2) einer Suzuki-Reaktion mit einer Verbindung der folgenden Formel (3), um eine Verbindung der folgenden Formel (4) zu erhalten;
(ii) Entschützen der Verbindung der folgenden Formel (4), um eine Verbindung der folgenden Formel (5) zu erhalten; und
(iii) Umsetzen der Verbindung der folgenden Formel (5) mit einer Guanidin-Verbindung: 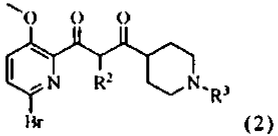 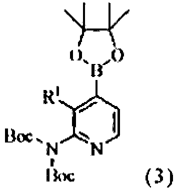 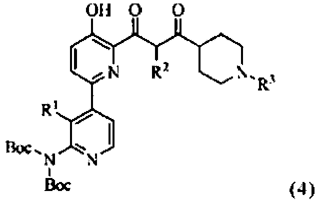 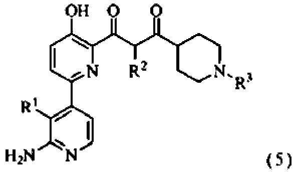 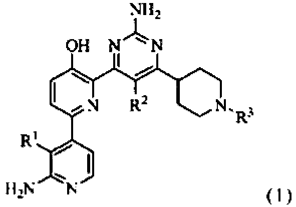 wobei,
R¹ Halogen ist,
R² Wasserstoff oder eine C1-C6-Alkylgruppe ist, und
R³ eine C1-C6-Alkylgruppe ist.

2. Verfahren nach Anspruch 1, wobei die Suzuki-Reaktion des Schritts (i) in Gegenwart von Tetrakis(triphenylphosphin)palladium(0) und Kaliumphosphat durchgeführt wird.

3. Verfahren nach Anspruch 1, wobei das Entschützen in Schritt (ii) in Gegenwart von Trifluoressigsäure durchgeführt wird.

4. Verfahren nach Anspruch 1, wobei die Guanidinverbindung aus Schritt (iii) Guanidinhydrochlorid ist.

5. Verfahren nach Anspruch 1, wobei die Reaktion des Schritts (iii) in Gegenwart von Kaliumcarbonat in Pyridin Lösungsmittel durchgeführt wird.

6. Verfahren nach Anspruch 1, umfassend den Schritt des Reinigens der aus Schritt (iii) erhaltenen Pyrimidinyl-Bipyridin-Verbindung der Formel (1) mit Aceton und Ethanol.

7. Verfahren nach einem der Ansprüche 1 bis 6, umfassend den weiteren Schritt der Umsetzung der Verbindung der Formel (1) mit einer Säure unter Bildung eines pharmazeutisch verträglichen Salzes der Verbindung der Formel (1).

8. Verfahren nach Anspruch 7, wobei die Säure Salzsäure ist und das pharmazeutisch verträgliche Salz der Verbindung der Formel (1) das Trihydrochloridsalz der Verbindung der Formel (1) ist.

9. Verfahren nach Anspruch 8, umfassend den Schritt des Rekristallisierens des Trihydrochloridsalzes der Verbindung der Formel (1) mit Wasser und Aceton.

10. Verbindung der folgenden Formel (4): 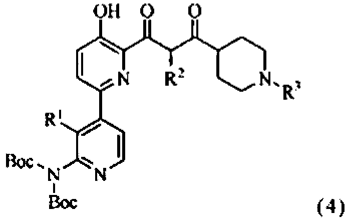 wobei,
R¹ e Halogen ist,
R² Wasserstoff oder eine C1-C6-Alkylgruppe ist, und
R³ eine C1-C6-Alkylgruppe ist.

11. Verbindung der folgenden Formel (5): 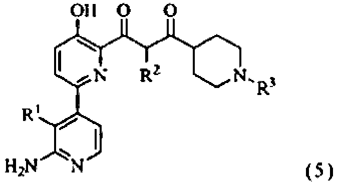 wobei,
R¹ Halogen ist,
R² Wasserstoff oder eine C1-C6-Alkylgruppe ist, und
R³ eine C1-C6-Alkylgruppe ist.

12. Verbindung nach Anspruch 10 oder 11, wobei R¹ Fluor ist, R² eine Methylgruppe ist und R³ eine *i*-Propylgruppe ist.

## Revendications

1. Procédé de préparation d'un composé pyrimidinylbipyridine de la formule (1) suivante, ledit procédé comprenant les étapes consistant à :
(i) soumettre un composé de la formule (2) suivante à une réaction de Suzuki avec un composé de la formule (3) suivante pour obtenir un composé de la formule (4) suivante ;
(ii) déprotéger le composé de la formule (4) suivante pour obtenir un composé de la formule (5) suivante ; et
(iii) faire réagir le composé de la formule (5) suivante avec un composé de guanidine : 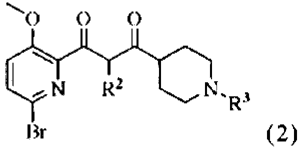 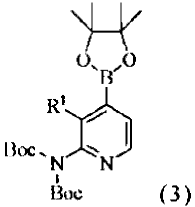 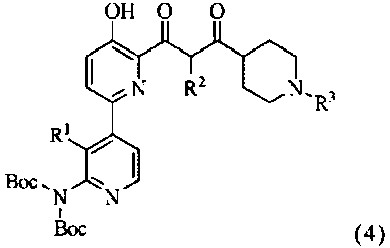 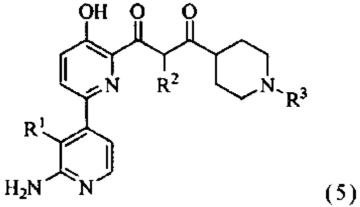 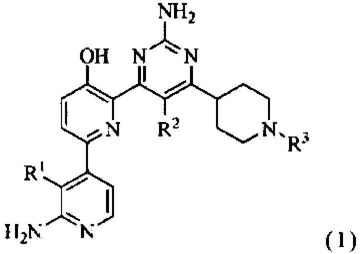 dans laquelle
R¹ est un halogène,
R² est un atome d'hydrogène ou un groupe alkyle en C₁-C₆, et
R³ est un groupe alkyle en C₁-C₆.

2. Procédé selon la revendication 1, dans lequel la réaction de Suzuki de l'étape (1) s'effectue en présence de tétrakis(triphénylphosphine)palladium(0) et de phosphate de potassium.

3. Procédé selon la revendication 1, dans lequel la déprotection de l'étape (ii) s'effectue en présence d'acide trifluoroacétique.

4. Procédé selon la revendication 1, dans lequel le composé de guanidine de l'étape (iii) est le chlorhydrate de guanidine.

5. Procédé selon la revendication 1, dans lequel la réaction de l'étape (iii) s'effectue en présence de carbonate de potassium dans un solvant pyridine.

6. Procédé selon la revendication 1, comprenant l'étape de purification du composé pyrimidinylbipyridine de formule (1) obtenu à partir de l'étape (iii) avec de l'acétone et de l'éthanol.

7. Procédé selon l'une quelconque des revendications 1 à 6, comprenant l'étape supplémentaire consistant à faire réagir le composé de formule (1) avec un acide pour former un sel pharmaceutiquement acceptable du composé de formule (1).

8. Procédé selon la revendication 7, dans lequel l'acide est l'acide chlorhydrique et le sel pharmaceutiquement acceptable du composé de formule (1) est le sel trichlorhydrate du composé de formule (1).

9. Procédé selon la revendication 8, comprenant l'étape de recristallisation du sel trichlorhydrate du composé de formule (1) avec de l'eau et de l'acétone.

10. Composé de la formule (4) suivante : 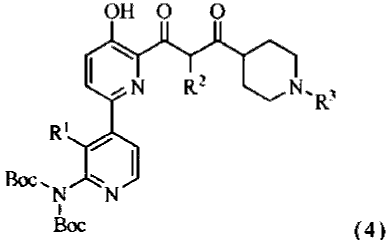 dans laquelle
R¹ est un halogène,
R² est un atome d'hydrogène ou un groupe alkyle en C₁-C₆, et
R³ est un groupe alkyle en C₁-C₆.

11. Composé de la formule (5) suivante : 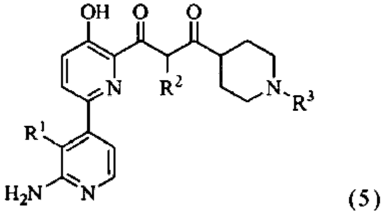 dans laquelle
R¹ est un halogène,
R² est un atome d'hydrogène ou un groupe alkyle en C₁-C₆, et
R³ est un groupe alkyle en C₁-C₆.

12. Composé selon la revendication 10 ou la revendication 11, dans lequel R¹ est un atome de fluor, R² est un groupe méthyle et R³ est un groupe isopropyle.
